# EUROPEAN PATENT SPECIFICATION

(11) **EP 4 112 116 B1**
(45) Date of publication and mention of the grant of the patent: **12.11.2025**
(21) Application number: 21199067.6
(22) Date of filing: 27.09.2021
(51) Int. Cl.: A61N 1/05, A61N 1/06, A61N 1/36, A61N 5/06

(54) **IMPLANTABLE STIMULATOR**
IMPLANTIERBARER STIMULATOR
STIMULATEUR IMPLANTABLE

(30) Priority: 28.06.2021 US 202117360609
(43) Date of publication of application: 04.01.2023
(73) Proprietor: Painlab Ltd., London EC2V 6DL (GB)
(72) Inventor: Yearwood, Thomas, London (GB); Ward, Stephen Patrick, London (GB)
(74) Representative: Derry, Paul Stefan

(56) References cited:
- US-A1- 2008 140 149
- US-A1- 2013 317 572
- US-A1- 2016 220 842
- US-A1- 2020 155 854

## Description

### BACKGROUND

Electrical stimulation can be applied to nervous or other tissues to alleviate pain or to provide other medical benefits. Such electrical stimulation can be provided by fully implantable stimulator systems, which can include a pulse generator unit and one or more flexible leads attached thereto. Such lead(s) can include array(s) of electrical contacts (electrodes) that can be used to deliver pulses of electrical stimulus to neurological or other tissue. To provide stimulation to tissue of the spinal cord, such stimulator leads can be installed percutaneously, via a needle inserted into the epidural space and then 'steered' into position within the epidural space using a stylet. Additionally or alternatively, a 'paddle' style stimulator lead can be implanted surgically by way of an open laminotomy. To stimulate peripheral nervous tissue, a stimulator lead may be positioned adjacent to the peripheral nerve or other peripheral neurological tissue percutaneously or via open surgical techniques. Once implanted, a pulse generator, which can be positioned subcutaneously in a location position distant from the end of a stimulator lead, can apply electrical energy via the electrodes of the stimulator lead. This delivered electrical energy (e.g., time-varying electrical fields, injected electrical currents, etc.) in turn can modify the function of the nervous system in order to alleviate chronic pain, reduce tremor, facilitate locomotor rehabilitation following injury, etc.

US 2013/317572 A1 discloses a method of treating a patient with an ailment using an optical element implanted within the patient, comprising conveying low-level laser energy having a wavelength in the range of 600 nm-2500 nm from the optical element to a neuronal element of the patient, thereby modulating the neuronal element to treat the ailment.

### SUMMARY

The claims define the matter for protection. In a first aspect, an implantable stimulator is provided that is configured to be secured proximate to a target tissue, the stimulator comprising: (i) an elongate cylindrical lead body, wherein the lead body comprises a first plurality of cylindrical segments that alternate with a second plurality of cylindrical segments along a long axis of the lead body; (ii) a plurality of electrodes, wherein each electrode of the plurality of electrodes forms at least part of an external surface of a respective one of the first plurality of cylindrical segments of the lead body; and (iii) a plurality of light emitters, wherein each light emitter of the plurality of light emitters is disposed within a respective one of the second plurality of cylindrical segments of the lead body, and wherein each light emitter of the plurality of light emitters is configured to emit light at at least one specified wavelength.

In some examples of the first aspect, at least part of an outer surface of a given one of the second plurality of cylindrical segments is formed from a transparent flexible material that at least partially encloses a light emitter of the plurality of light emitters that is disposed within the given one of the second plurality of cylindrical segments. For example, the transparent flexible material can include poly(methyl methacrylate). Additionally or alternatively, the transparent flexible material can have a cylindrical shape and all of the outer surface of the given one of the second plurality of cylindrical segments can be formed from the transparent flexible material.

In some examples of the first aspect, the implantable stimulator additionally includes a transverse element, wherein the transverse element is disposed within one of the first plurality of cylindrical segments, and wherein the transverse element has a central aperture shaped to permit passage of a stylus. For example, the transverse element can also have a plurality of peripheral apertures disposed around the central aperture, and the implantable system can additionally include a wire that is connected to one of the light emitters and that passes through one of the peripheral apertures.

In some examples of the first aspect, the elongate cylindrical lead body has an outer circumference between 1 millimeter and 2.5 millimeters and a total length between 25 centimeters and 90 centimeters, wherein at least one of the first plurality of cylindrical segments has a length between 2 millimeters and 5 millimeters, and wherein at least one of the second plurality of cylindrical segments has a length between 3 millimeters and 6 millimeters.

In some examples of the first aspect, a first light emitter and a second light emitter of the plurality of light emitters are located proximate to each other within the elongate cylindrical lead body such that light emitted from the first light emitter constructively and destructively interferes with light emitted from the second light emitter, thereby enhancing the optical excitation of a focal volume within a portion of the target tissue while reducing the optical excitation of one or more other volumes within the portion of target tissue when the stimulator is proximate to the target tissue.

In some examples of the first aspect, the implantable stimulator additionally includes a controller, wherein the controller includes one or more processors configured to perform controller operations comprising: (a) operating a first light emitter of the plurality of light emitter to emit light at the at least one specified wavelength to provide optical excitation to a portion of the target tissue; and (b) operating the first electrode to provide electrical excitation to the portion of the target tissue at the same time that the first light emitter is emitting light.

In some examples of the first aspect, the implantable stimulator additionally includes a controller, wherein the controller includes one or more processors configured to perform controller operations comprising: (a) operating the first electrode to provide electrical excitation to a portion of the target tissue; and (b) subsequent to operating the first electrode to provide electrical excitation, operating the first light emitter to emit light at the at least one specified wavelength to provide optical excitation to the portion of the target tissue.

In some examples of the first aspect, at least one light emitter of the plurality of light emitters is configured to emit non-coherent light at the at least one specified wavelength. For example, the at least one light emitter can include a superluminescent diode.

In some examples of the first aspect, the at least one specified wavelength includes a wavelength that is between 600 nanometers and 700 nanometers or between 770 nanometers and 1200 nanometers.

In a second aspect, an implantable stimulator is provided that is configured to be secured proximate to a target tissue, the stimulator comprising: (i) an elongate cylindrical lead body, wherein the lead body comprises a first plurality of cylindrical segments that alternate with a second plurality of cylindrical segments along a long axis of the lead body; (ii) a plurality of light emitters, wherein each light emitter of the plurality of light emitters is disposed within a respective one of the second plurality of cylindrical segments of the lead body, and wherein each light emitter of the plurality of light emitters is configured to emit light at at least one specified wavelength, and wherein at least part of an outer surface of a given one of the second plurality of cylindrical segments is formed from a transparent flexible material that at least partially encloses a light emitter of the plurality of light emitters that is disposed within the given one of the second plurality of cylindrical segments; and (iii) a transverse element, wherein the transverse element is disposed within one of the first plurality of cylindrical segments, and wherein the transverse element has a central aperture shaped to permit passage of a stylus.

In some examples of the second aspect, the transparent material includes poly(methyl methacrylate).

In some examples of the second aspect, the transparent flexible material has a cylindrical shape and all of the outer surface of the given one of the second plurality of cylindrical segments is formed from the transparent flexible material.

In some examples of the second aspect, the transverse element also has a plurality of peripheral apertures disposed around the central aperture, and the implantable system additionally includes a wire that is connected to one of the light emitters and that passes through one of the peripheral apertures.

In some examples of the second aspect, a first light emitter and a second light emitter of the plurality of light emitters are located proximate to each other within the elongate cylindrical lead body such that light emitted from the first light emitter constructively and destructively interferes with light emitted from the second light emitter, thereby enhancing the optical excitation of a focal volume within a portion of the target tissue while reducing the optical excitation of one or more other volumes within the portion of target tissue when the stimulator is proximate to the target tissue.

In a third aspect, an implantable stimulator is provided that is configured to be secured proximate to a target tissue, the stimulator comprising: (i) a paddle-shaped lead body having a first side and a second side; (ii) a plurality of electrodes, wherein each electrode of the plurality of electrodes is disposed at a respective location on the first side of the lead body; and (iii) a plurality of light emitters, wherein each light emitter of the plurality of light emitters is configured to emit light at at least one specified wavelength from the first side of the lead body, and wherein the plurality of light emitters are disposed within the lead body relative to the plurality of electrodes such that a portion of the target tissue can receive optical excitation from at least one of the light emitters and electrical excitation from at least one of the electrodes.

In some examples of the third aspect, the electrodes are arranged in pairs of electrodes along a long axis of the lead body, and the pairs of electrodes alternate with the light emitters along the long axis of the lead body.

In some examples of the third aspect, each pair of electrodes includes first and second electrodes that have mirrored locations and shapes about the long axis of the lead body

The foregoing summary is illustrative only and is not intended to be in any way limiting. In addition to the illustrative aspects, embodiments, and features described above, further aspects, embodiments, and features will become apparent by reference to the figures and the following detailed description and the accompanying drawings.

### BRIEF DESCRIPTION OF THE FIGURES

Figure 1 illustrates aspects of an example device implanted in a body.
Figure 2A illustrates aspects of an example stimulator.
Figure 2B illustrates aspects of an example stimulator.
Figure 2C illustrates aspects of an example stimulator.
Figure 2D illustrates aspects of an example stimulator.
Figure 2E illustrates aspects of an example stimulator.
Figure 2F illustrates aspects of an example stimulator.
Figure 3 illustrates aspects of an example stimulator.
Figure 4 illustrates aspects of an example stimulator.
Figure 5 illustrates aspects of an example system.

### DETAILED DESCRIPTION

The following detailed description describes various features and functions of the disclosed systems and methods with reference to the accompanying figures. The illustrative system and method embodiments described herein are not meant to be limiting. It may be readily understood that certain aspects of the disclosed systems and methods can be arranged and combined in a wide variety of different configurations, all of which are contemplated herein.

### I. Overview

Monochromatic light can be provided to a variety of tissues to provide a variety of benefits, including improved wound healing and pain management or other neuromodulatory effects. However, it can be difficult to provide light of sufficient intensity at the necessary wavelength(s) to target tissues, especially where those tissues are deep, within bone, etc. (e.g., to tissues of the spinal cord or dorsal root ganglia). These difficulties are compounded by the need to provide such stimulation from a device that can be easily and reliably surgically implanted and interfaced with an implanted pulse generator unit and/or other implanted systems.

It is also beneficial to provide electrical stimulation and optical stimulation to tissue simultaneously, alternatingly, or according to some other protocol. However, the difficulties above in providing high-power, monochromatic optical stimulation to a target tissue are further compounded by the added requirement that a device also provide electrical stimulation, especially where it is desired that the device be capable of providing electrical and optical stimulation to the *same* tissue.

Embodiments described herein provide a variety of devices for providing such high-power monochromatic optical stimulation and/or collocated electrical stimulation to deep nervous or other tissues in a manner that is compatible with existing implanted systems and surgical implantation methods and tools. These embodiments include incorporating superluminescent LEDs (SLEDs) or other small, high-power, high-efficiency monochromatic or otherwise configured light emitters into thin, narrow, or otherwise appropriately dimensioned stimulators. This allows the stimulators to be accurately implanted into clinically relevant anatomy (e.g., through natural or surgically-formed passages into the lumen of the spinal column to permit stimulation of the spinal cord).

Additionally, placing appropriate light-emitting elements within the stimulator allows the light emitted from the light emitters to be delivered directly to the target tissue. This avoids the use of optical fibers or other means for coupling light from more remote light emitters (e.g., lasers located in a pulse generator that is distant from the stimulator) to the target tissue. Thus, the present embodiments provide improved efficiency and reliability while avoiding complicated routing, assembly, and minimum bend radius constraints that can be associated with the use of optical fibers.

Electrical power can be provided to the light emitters in such a stimulator using wiring similar to that used in contemporary stimulators (e.g., multi-electrode cylindrical catheter-type stimulators) to provide electrical signals to electrodes. Thus, reliable fabrication methods used to assemble prior electrical stimulators can be applied to fabricate the optical or combined optical/electrical stimulators described herein. This can lead to reduced development and fabrication costs, eased regulatory certification, increased reliability, and other benefits.

Electrical stimulation of the spinal cord can be used to treat control chronic pain (Joosten, E. A. and Franken, G. (2020) 'Spinal cord stimulation in chronic neuropathic pain: mechanisms of action, new locations, new paradigms', Pain. NLM (Medline), 161(1), pp. S104-S113). It had been hypothesized that this electrical treatment modulates pain transmission by manipulating a gating mechanism within the dorsal columns of the spinal cord, this gating mechanism having been proposed by Melzack and Wall in 1965 (Melzack, R. and Wall, P. D. (1965) 'Pain mechanisms: A new theory', Science. Science, 150(3699), pp. 971-979). The first implantable spinal cord electrical stimulator device for chronic pain was implanted in 1971 (Shealy, C. N., Mortimer, J. T. and Hagfors, N. R. (1970) 'Dorsal column electroanalgesia.', Journal of neurosurgery. J Neurosurg, 32(5), pp. 560-564). However, electrical stimulation for the treatment of chronic pain rarely provides complete abolition of chronic pain, with clinical 'success' defined as an average improvement in pain intensity of 50%. This level of success is seen in approximately 67-100% of patients in the period of 12 months or less following implantation and in only 46-76% of patients thereafter (Baranidharan, G. et al. (2021) 'Efficacy and safety of 10 kHz spinal cord stimulation for the treatment of chronic pain: A systematic review and narrative synthesis of real-world retrospective studies', Biomedicines. MDPI AG, 9(2), pp. 1-21).

Photobiomodulation (PBM) is the application of monochromatic light through the skin with a low energy density to induce non thermal photochemistry effects at a cellular level (Desmet, K. D. et al. (2006) 'Clinical and experimental applications of NIR-LED photobiomodulation', Photomedicine and Laser Surgery. Photomed Laser Surg, pp. 121-128). Previously termed Low Level Laser Light Therapy, PBM was discovered in 1967 by Endre Mester at the Semmelweis University in Hungary. Mester investigated the effects of low powered laser light on malignant tumours in rodents and observed a heightened rate of hair growth and accelerated wound healing (Mester, Adam and Mester, Andrew (2017) 'The History of Photobiomodulation: Endre Mester (1903-1984)', Photomedicine and Laser Surgery. Mary Ann Liebert Inc., pp. 393-394). PBM has developed as a clinical treatment that involves the application of various wavelengths of monochromatic light through the skin to achieve a biological effect on a target tissue beneath the skin. The light source is placed near or in contact with the skin to such that light emitted therefrom penetrates tissue and leads to molecular and cellular changes (Cheng, K. et al. (2021) 'Mechanisms and Pathways of Pain Photobiomodulation: A Narrative Review', The Journal of Pain. Elsevier BV. doi: 10.1016). PBM is limited in that light must penetrate the skin to achieve an effect. Gursoy and Bradley in 1996 showed that the light wavelength frequently used in PBM devices (near infra-red 830nm) was only able to penetrate up to 5cm beneath the skin surface (Gursoy B and Bradley PF (no date) 'A study of soft tissue penetration by 830 nm wavelength using radiometer and CCD camera.', Proceedings of the International Congress on Lasers in Dentistry. London p. 105). Photobiomodulation has the capacity to modulate pain (Cheng *et al.*, 2021). Light stimulation does not induce paraesthesia or have motor effects. Light stimulation at red and near infra-red wavelengths is not carcinogenic or mutagenic.

A variety of mechanisms have been proposed to explain the physiological effects of PBM. Mitochondria contain chromophores which absorb photons from PBM. The primary chromophore to absorb red and near-infrared light is the enzyme cytochrome c oxidase (CCO), which is located at unit IV of the mitochondrial respiratory chain. This endogenous photoreceptor, when stimulated by red light, is up-regulated and activates light-altered signalling pathways. Red (600-700nm) and near-infrared (770-1200nm) light at low irradiance excites cytochrome c oxidase and promotes electron transport. Longer wavelengths (>900nm) and wavelengths in the blue and green light range may influence calcium channels. Up-regulating CCO concentration enhances the capacity for cellular oxygen metabolism. Because neurons are highly dependent on oxygen metabolism, this mechanism results in metabolic and haemodynamic alterations that modify neuronal functioning (Cheng *et al.*, 2021). PBM has been shown to disrupt fast axonal transport by causing perturbation of microtubule arrays of small diameter neurons in rat dorsal root ganglia in culture and by reducing ATP synthesis in axonal mitochondria (Chow, R. T. and Armati, P. J. (2016) 'Photobiomodulation: Implications for anesthesia and pain relief', Photomedicine and Laser Surgery. Mary Ann Liebert Inc., 34(12), pp. 599-609). PBM using 830 nm irradiation specifically suppresses in vivo nerve conduction in small diameter, thinly myelinated A_{d} and unmyelinated C fibres that have been electrically stimulated (Tsuchiya, K. et al. (1994) 'Laser irradiation abates neuronal responses to nociceptive stimulation of rat-paw skin', Brain Research Bulletin. Brain Res Bull, 34(4), pp. 369-374). 830 nm laser irradiation of rat DRG cultures induces the formation of static axonal varicosities in small and medium diameter, TRPV-1 positive neurons, which resolve after 24 hrs. Laser irradiation also induces a progressive and statistically significant decrease in mitochondrial membrane potential in and between the axonal varicosities where the mitochondria are seen as clusters (Holanda, V. et al. (2018) 'The Mechanistic Basis for Photobiomodulation Therapy of Neuropathic Pain by Near Infrared Laser Light', Arquivos Brasileiros de Neurocirurgia: Brazilian Neurosurgery. John Wiley and Sons Inc., 37(04), pp. 317-325). In a mouse sciatic nerve crush model, PBM reduced mechanical hypersensitivity and decreased spinal cord and sciatic nerve levels of TNFa (Cidral-Filho, F. J. et al. (2013) 'Light-emitting diode therapy induces analgesia and decreases spinal cord and sciatic nerve tumour necrosis factor - A levels after sciatic nerve crush in mice', European Journal of Pain (United Kingdom). Eur J Pain, 17(8), pp. 1193-1204). PBM treated animals exhibit a decrease in pro-inflammatory markers (tumor necrosis factor (TNF), interleukin 1β(IL-1β), and hypoxia-inducible factor 1-α(HIF-1 a)) at a chronic constriction injury site in the rat sciatic nerve when compared with non-treated-injured animals (Hsieh, Y. L. et al. (2012) 'Low-level laser therapy alleviates neuropathic pain and promotes function recovery in rats with chronic constriction injury: Possible involvements in hypoxia-inducible factor 1α (HIF-1α)', Journal of Comparative Neurology. J Comp Neurol, 520(13), pp. 2903-2916).

Holanda et al delivered 808nm laser light to the dorsal root ganglia of patients with chronic axial low back pain through a needle positioned in the 2^{nd} lumbar intervertebral foramen. 70% of the patients treated with laser light reported 50% pain relief at one month post treatment (Holanda, V. M. et al. (2016) 'Photobiomodulation of the dorsal root ganglion for the treatment of low back pain: A pilot study', Lasers in Surgery and Medicine. John Wiley and Sons Inc., 48(7), pp. 653-659).

Delivery of a subthreshold electrical stimulus concurrently with infrared light lowered the required optical energy per pulse to achieve stimulation and delivering a subthreshold electrical stimulus to enhance the excitability of neural tissue to optical stimuli occurs in a nonlinear fashion (Duke, A. R. et al. (2009) 'Combined optical and electrical stimulation of neural tissue in vivo', Journal of Biomedical Optics. SPIE-Intl Soc Optical Eng, 14(6), p. 060501).

A stimulator as described herein could have a cylindrical geometry, a paddle-shaped geometry, or some other geometry appropriate to providing optical or combined electrical and optical stimulation to a target tissue and to surgically implanting the stimulator in position to provide such stimulation. As described in detail below, the target tissue could be tissue of the spinal cord, spinal nerves, dorsal root ganglia, or other nervous tissue associated with the spinal cord. However, the stimulators described herein could alternatively be used and/or adapted to provide stimulus to peripheral nerves, cranial nerves, nervous tissue of the brain, mesenteric nervous tissue, or some other nervous tissue. Additionally or alternatively, the stimulators described herein could be used and/or adapted to provide stimulus to muscle or other non-nervous tissues.

Additionally, a stimulator as described herein could be used as part of a fully-implanted stimulation system. For example, such a stimulator could include connectors to facilitate electrical connection to an implantable pulse generator and/or some other implantable system and/or to a cable that could be configured to electrically couple the stimulator to such an implantable system. In another example, such a stimulator could be provided already connected to (e.g., assembled as part of) an implantable system that include pulse generator, cabling, and/or other components. Alternatively, a stimulator as described herein could be used as part of a percutaneous stimulator system, with cabling or other means electrically connecting the wholly or partially implanted stimulator to a pulse generator or other components located outside the body. In some examples, such a stimulator could be configured to be implanted and initially operated using a non-implanted system (e.g., an extra-corporeal clinical stimulus generator connected to the stimulator via transcutaneous cabling) to determine optimal positioning and stimulus parameters. Once the clinical efficacy, optimal stimulus parameters, or other information about the stimulator is determine using the non-implanted system, the stimulator could then be disconnected from the non-implanted system and coupled to a fully-implanted system (e.g., programmed using the information generated using the non-implanted system).

The embodiments described herein are particularly adapted to providing optical or combined electrical and optical stimulation to tissue of the spinal cord, dorsal root ganglia, and peripheral nerves in order to inhibit pain or to provide other neuromodulatory effects. Conventional electrical stimulation may be supplemented by stimulation with light from single or multiple light sources contained within the same stimulator (e.g., lead or paddle array). Alternatively, such a stimulator could lack electrodes and could operate, using light emitters located within the stimulator, to provide optical stimulation alone.

Although example implementations are described below with reference to modulating the dorsal column, dorsal horn, dorsal root, dorsal root entry zone, and/or other particular regions of the spinal column to control pain, the modulation may in some instances be directed to other neurological structures and/or target neural populations of the spinal cord and/or other neurological tissues. Some implementations can have configurations, components, or procedures different than those described in this section, and other implementations may eliminate particular components or procedures.

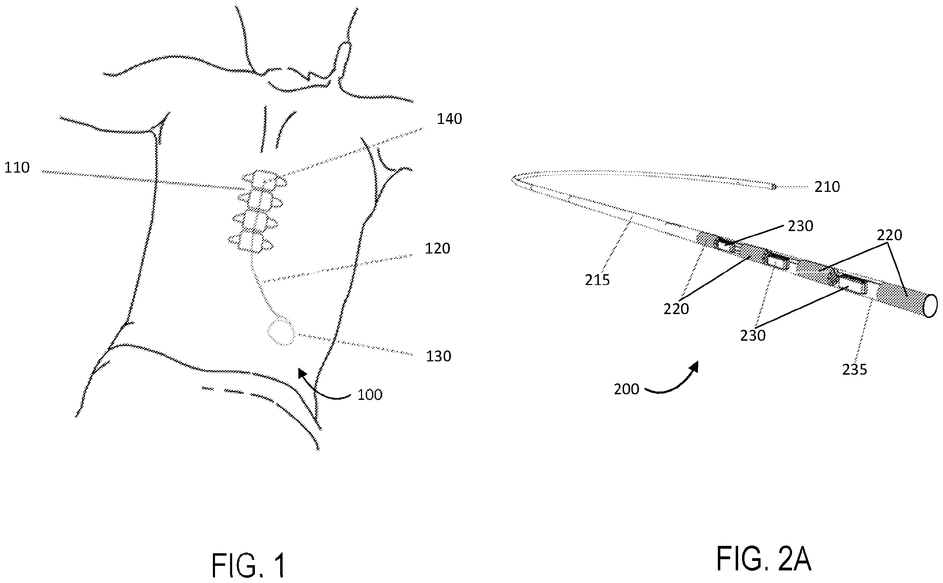

Fig. 1 depicts an example implantable stimulator 100 relative to the general anatomy of a patient's spinal column 110. The stimulator 100 can include a pulse generator 130 implanted subcutaneously within a patient. The pulse generator 130 is coupled, via a lead 120, to a stimulator 140 that is implanted within the spinal column proximate to tissue of the spinal cord, dorsal root ganglion, spinal nerve, or some other target tissue. The stimulator 140 could bon continuous with the lead 120 or could be coupled thereto via a connector or other coupling means. The stimulator 140 could include an array of light emitters and/or an array of electrodes to provide optical and/or electrical stimulus to the target tissue.

As noted above, it is beneficial to provide active light-emitting elements (e.g., SLEDs or other elements configured to provide substantially monochromatic or otherwise spectrally specified light) within a stimulator (e.g., 140) in order to provide therapeutic light directly to target tissue. This avoids the negative aspects of providing light from outside the body and/or using optical fibers to couple light from a distant source (e.g., a laser located in the pulse generator 130) to the target tissue via the stimulator 140. It is also beneficial that these light emitters have size, power, efficiency, and other parameters that are compatible with incorporation into standard-sized cylindrical catheter-style stimulators (e.g., that also include central lumens for the use of stylets to guide insertion of the stimulators), paddle-style stimulators, or other geometries commonly used to provide electrical stimulus to target tissues. This allows existing surgical techniques and instruments to be applied to implant these improved stimulators in the sort of anatomical sites commonly used for electrical stimulation.

A variety of small, high-power, high-efficiency light emitting elements, including a variety of light-emitting diodes (LEDs), have recently become available that satisfy the above operational parameters. These LED light sources have suitably small sizes (e.g., a largest dimension less than 1mm) to be incorporated into conventional stimulator geometries and suitably high emitted light powers at the desired wavelength (e.g., greater than 1 J/cm²) to provide clinically relevant optical stimulation to neurological tissue or other tissue of interest. Such LED sources are also superior to the laser sources traditionally employed in non-implanted PBM systems in that they have lower current requirements and higher efficiencies while still providing substantially monochromatic light, allowing these LED sources to be driven by circuitry of implantable pulse generators, which can be limited with respect to maximum drive current, instantaneous power output, and total stored energy.

Suitable LED light emitters include conventional LEDs, organic LEDs (OLEDs), perovskite LEDs (PLEDs), and superluminescent LEDs (SLEDs). An example of a suitable commercially available light emitter is the Osram Firefly SFH 4055, which exhibits a peak wavelength at 860nm and a radiant intensity of 40 mW/sr while having dimensions of 1.0mm x 0.55mm x 0.325mm. Indeed, these light emitters are now capable of sufficiently narrow emission spectra that a stimulator as described herein could include two (or more) different light emitters that emit light at slightly different emission wavelengths such that the light from the two (or more) light emitters constructively and destructively interfere. The locations, orientations, wavelengths, and emission profiles of these two (or more) light emitters could then be specified within a stimulator such that the pattern of constructive and destructive interference enhances the excitatory or other clinical effect of the emitted light on tissue within certain volume(s) relative to the stimulator. For example, such that light emitted from a first light emitter constructively and destructively interferes with light emitted from a second light emitter, thereby enhancing a magnitude of optical excitation of a focal volume within a portion of a target tissue that is proximate to the first and second light emitters while reducing a magnitude of optical excitation of one or more other volumes within the portion of the target tissue.

As noted above, the application of LED light stimulation (e.g., at red and near-infrared wavelengths) and electrical stimulation to the same neurological tissue (e.g., simultaneous and/or sequentially) may be synergistic, providing greater clinical benefits than either type of stimulation alone. For example, a patient can receive effective pain relief by the combination of LED light stimulation and electrical stimulation by virtue of a synergistic mechanism. The patient can receive effective pain relief by the addition of LED light stimulation to electrical stimulation by targeting additional pain relieving mechanisms at a cellular level and/or by enhancement of the effect of the mechanism of the electrical stimulus due to the optical stimulation increasing the degree of effect of the electrical stimulus on the tissue. The small size and location within the stimulator of the light emitters described herein allows those light emitters to target the same, small target tissues with both electrical and optical stimulus. As described in greater detail below, this can be done by disposing the light emitters and electrodes alternatingly along the axis of a cylindrical catheter-type stimulator or by disposing the electrodes and light emitters near each other on/within a stimulator according to some other pattern.

The implementations described herein are predominantly described in the context of treating chronic neuropathic spinal pain and radicular pain with electrical and light modulation applied to the spinal cord in the lower thoracic vertebral territory (T9-T12). However, clinically relevant modulation signals or other therapeutic stimulus can be applied to other patient locations to address other indications. For example, optical and/or electrical stimulus may be applied to the dorsal root ganglia and foramen region laterally outward from the center of the spinal cord/spinal column. Stimulus may be applied to other spinal levels including the sacrum (e.g., to treat urinary or faecal incontinence) or above the thoracic territory (e.g., to treat angina). Stimulus may be applied to the cervical region to address radicular pain, complex regional pain and/or total body pain. Stimulation may be applied to the occipital nerves, for example, in the treatment of migraine.

As noted above, the stimulators described herein could be used to provide optical and/or electrical stimulus to a variety of target tissues in order to address a variety of symptoms or other clinically relevant issues. For example, a stimulator as described herein could be implanted to provide stimulus to spinal cord tissue in the C1-C5 region (e.g., C2-C3) in order to address one or more of a variety of headache pain syndromes, including but not limited to herpes zoster (shingles) (providing stimulus to C4-C5 and/or the trigeminal nerve), migraine, cluster headaches, swimmer's headaches, analgesic rebound headaches, and/or occipital neuralgia.

**In** some examples, a stimulator as described herein could be implanted to provide stimulus to spinal cord tissue in the C2-C4 region (e.g., C2-C3) in order to address one or more pain syndromes, including but not limited to trigeminal neuralgia, temporomandibular joint dysfunction, atypical facial pain, myofascial pain syndrome of the face, cancer pain, hyoid syndrome, and/or reflex sympathetic dystrophy of the face.

**In** some examples, a stimulator as described herein could be implanted to provide stimulus to spinal cord tissue in the C2-C6 region (e.g., C3-C4) in order to address one or more neck and/or brachial plexus pain syndromes, including but not limited to cervical facet syndrome, cervical radiculopathy , fibromyalgia of the cervical musculature, myofascial pain syndrome experienced in the cervical musculature, cervical strain, cervicothoracic interspinous bursitis syndrome, spinal stenosis, brachial plexopathy, Pancoast's syndrome, and/or thoracic outlet syndrome.

In some examples, a stimulator as described herein could be implanted to provide stimulus to spinal cord tissue in the C2-C6 region (e.g., C3-C5) in order to address one or more shoulder pain syndromes, including but not limited to arthritic shoulder pain, acromio-clavicular joint pain, shoulder fibromyalgia, shoulder myofascial pain syndrome, subdeltoid bursitis, bicipital tendonitis, supraspinatus syndrome, rotator cuff tear, deltoid syndrome, teres major syndrome, and/or scapulocostal syndrome.

In some examples, a stimulator as described herein could be implanted to provide stimulus to spinal cord tissue in the C2-C6 region (e.g., C3-C5) in order to address one or more elbow pain syndromes, including but not limited to arthritic elbow pain, tennis elbow, golfer's elbow, anconeus syndrome, supinator syndrome, brachioradialis syndrome, ulnar nerve entrapment at the elbow, lateral antebrachial cutaneous nerve syndrome, and/or olecranon bursitis.

In some examples, a stimulator as described herein could be implanted to provide stimulus to spinal cord tissue in the C2-C6 region (e.g., C3-C5) in order to address one or more other upper extremity pain syndromes, including but not limited to fibromyalgia-related arm pain, myofascial pain syndrome, and/or phantom limb pain.

In some examples, a stimulator as described herein could be implanted to provide stimulus to spinal cord tissue in the C2-C6 region (e.g., C3-C5) in order to address one or more wrist pain syndromes, including but not limited to arthritic wrist pain, carpal tunnel syndrome, De Quervain's tenosynovitis, and/or arthritic carpometacarpal joint pain.

In some examples, a stimulator as described herein could be implanted to provide stimulus to spinal cord tissue in the C2-C6 region (e.g., C3-C5) in order to address one or more hand pain syndromes, including but not limited to arthritic finger pain, trigger thumb, trigger finger, ganglion cysts of the wrist & hand, sesamoiditis of the hand, plastic bag palsy, carpal boss syndrome, and/or Dupuytren's contracture.

In some examples, a stimulator as described herein could be implanted to provide stimulus to spinal cord tissue in the T1-T12 region in order to address one or more chest wall pain syndromes, including but not limited to costosternal syndrome, manubriosternal joint pain, intercostal neuralgia, diabetic truncal neuropathy, Tietze's syndrome, precordial catch syndrome, pain related to fractured ribs, and/or post-thoracotomy pain.

In some examples, a stimulator as described herein could be implanted to provide stimulus to spinal cord tissue in the T1-T12 region in order to address one or more thoracic spine pain syndromes, including but not limited to acute herpes zoster expressing in the thoracic dermatome, cancer pain, costovertebral arthritis pain, post-herpetic neuralgia, spinal stenosis, and/or pain related to thoracic vertebral compression fractures.

In some examples, a stimulator as described herein could be implanted to provide stimulus to spinal cord tissue in the T6-T12 region in order to address one or more abdominal & groin pain syndromes, including but not limited to acute pancreatitis, cancer pain, chronic pancreatitis, ilioinguinal neuralgia, visceral pain (peritoneum, stomach, duodenum, intestine, colon, liver, spleen, pancreas, kidney, adrenal gland, appendix, gall bladder, etc.), post-vasectomy pain syndrome, genitofemoral neuralgia, and/or interstitial cystitis.

In some examples, a stimulator as described herein could be implanted to provide stimulus to spinal cord tissue in the T8-T12 region in order to address one or more lumbar spine & sacroiliac joint pain syndromes, including but not limited to lumbar musculature fibromyalgia, myofascial pain syndrome, lumbar radiculopathy, latissimus dorsi muscle syndrome, spinal stenosis, arachnoiditis, and/or sacroiliac joint pain.

In some examples, a stimulator as described herein could be implanted to provide stimulus to spinal cord tissue in the T12-L5 region in order to address one or more pelvic pain syndromes, including but not limited to osteitis pubis, cancer pain, gluteus maximus syndrome, visceral pain (pelvis, coccyx, ovaries, fallopian tubes, uterus, vulva, clitoris, perineum, urinary bladder, testicles, rectum, etc.), piriformis syndrome, ishiogluteal bursitis, levator ani syndrome, coccydynia, interstitial cystitis, and/or vulvodynia.

In some examples, a stimulator as described herein could be implanted to provide stimulus to spinal cord tissue in the T7-T12 region in order to address one or more hip & lower extremity pain syndromes, including but not limited to lower extremity musculature fibromyalgia, lower extremity myofascial pain syndrome, musculature pain, arthritic hip pain, snapping hip syndrome, restless leg syndrome, iliopectinate bursitis, ischial bursitis, meralgia paresthetica, phantom limb pain (e.g., by stimulating at t7-t10), and/or trochanteric bursitis.

In some examples, a stimulator as described herein could be implanted to provide stimulus to spinal cord tissue in the T8-T12 region in order to address one or more knee pain syndromes, including but not limited to arthritic knee pain, medial collateral ligament pain, jumper's knee syndrome, runner's knee syndrome, suprapatellar bursitis, prepatellar bursitis, superficial infrapatellar bursitis, deep infrapatellar bursitis, baker's cyst, and/or pes anserine bursitis.

In some examples, a stimulator as described herein could be implanted to provide stimulus to spinal cord tissue in the T8-T12 region in order to address one or more ankle pain syndrome, including but not limited to arthritic ankle pain, arthritic midtarsal joints, deltoid ligament strain, anterior tarsal tunnel syndrome, posterior tarsal tunnel syndrome, and/or Achilles tendonitis.

In some examples, a stimulator as described herein could be implanted to provide stimulus to spinal cord tissue in the T8-T12 region in order to address one or more foot pain syndromes and/or foot syndromes associated with pain, including but not limited to arthritic toe pain, bunion pain, gout, Morton's neuroma, plantar fasciitis, calcaneal spur syndrome, mallet toe syndrome, and/or hammer toe syndrome.

In some examples, a stimulator as described herein could be implanted to provide stimulus to spinal cord tissue in the C2-C4 region e.g., C3-C4) in order to address one or more whole body pain syndromes, including but not limited to fibromyalgia, cancer pain, and/or multiple-limb chronic regional pain syndrome.

### II. Example Stimulators

As noted above, it is beneficial to provide active light-emitting elements within a stimulator in order to provide therapeutic light directly to target tissue, thus avoiding the negative aspects of providing light from outside the body and/or using optical fibers to couple light from a distant source. It is also beneficial that these light emitters have size, power, efficiency, and other parameters that are compatible with incorporation into stimulators having geometries the same as or similar to existing implantable electrical stimulation devices. Appropriate light emitting elements include a variety of LEDs having suitably small sizes (e.g., a largest dimension less than 1mm) to be incorporated into conventional stimulator geometries and suitably high emitted light powers at the desired wavelength (e.g., greater than 1 J/cm²) to provide clinically relevant optical stimulation to neurological tissue or other tissue of interest. Such LED sources include organic LEDs (OLEDs), perovskite LEDs (PLEDs), superluminescent LEDs (SLEDs), and two-way LEDs that incorporate quantum dots. Such light emitting elements could be configured to emit light at one or more specified therapeutic wavelengths, including but not limited to ultraviolet wavelengths (e.g., 1-380 nm), visible light wavelengths (e.g., 380-750nm), near infrared wavelengths (e.g., 700-1300nm), and infrared wavelengths (e.g., at or greater than 1330nm)

Stimulators including such light emitters (e.g., linear or otherwise-configured arrays of such light emitters) could have narrow and/or thin geometries to permit them to be guided through naturally-existing or surgically-formed holes or other features of bones to access a variety of target tissues (e.g., tissue of the spinal cord, dorsal root ganglia, etc.). For example, such stimulators could have a cylindrical or other catheter-style geometry. In another example, such stimulators could have a paddle-style geometry, with light emitters and/or electrodes distributed across one side of the paddle. Such a paddle-style stimulator could be installed on the surface of the spinal cord following, e.g., laminectomy to expose the spinal cord. Electrodes could also be included as part of such stimulators, e.g., alternating with the light emitters to allow electrical stimulation and optical stimulation to be applied to the same small regions of target tissue.

Fig. 2A depicts elements of an example catheter-style stimulator 200. The stimulator 200 can be connected to a pulse generator or other source of electrical power and signals via a lead 110 that is continuous with the stimulator. Electrical current is delivered by such a device via the 210 to electrical wires contained within a lead body 215. The current is supplied to multiple cylindrical metallic electrodes 220 and to multiple light emitting diodes 230 housed within transparent portions 235 of the stimulator 200. Alternatively, the stimulator 200 could be operated in an optical stimulus-only mode. In such examples, the metallic electrodes 220 could be disconnected from any wires or other conductors. Indeed, in such examples the segments between the transparent portions 235 of the stimulator 200 could be composed of non-metallic and/or non-conductive materials.

As depicted in Fig. 2A, the light emitters 230 are disposed within cylindrical segments 235 of the stimulator 200 that are entirely transparent. However, alternative configurations wherein only a portion of the segments containing the light emitters 230 (e.g., small rectangular or otherwise-shaped windows) are transparent. Additionally or alternatively, the stimulator 200 could include prisms, lenses, mirrors, diffraction gratings, filters, light spreaders, or other elements to shape the profile and/or spectrum of light emitted from the light emitters 230 (e.g., to ensure that light is emitted in only one direction from the stimulator 200, or to ensure that light is emitted omnidirectionally from the light emitter). The transparent elements 235 of the stimulator 200 could be composed of a semi-flexible material with very low absorbency for red/near infrared light or some other wavelength(s) of light of interest. For example, the transparent elements 235 could be composed of poly(methyl methacrylate) (PMMA).

As shown in Fig. 2A, each transparent segment 235 of the stimulator 200 contains a single light emitter 230. However, each transparent segment 235 could contain more than one light emitter. This could be done, e.g., to increase the amount of light emitted and/or to broaden and/or control an angle, relative to the stimulator 200, across which light is emitted. Additionally or alternatively, multiple light emitters of the same segment could emit light in the same direction but could differ with respect to wavelength of emitted light, location, orientation, or other parameters such that the light emitted from the different light emitters constructively and destructively interferes. This could be done such that the pattern of constructive and destructive interference enhances the excitatory or other clinical effect of the emitted light on tissue within certain volume(s) relative to the stimulator 200, e.g., to specifically target tissue at a specified depth within a target tissue.

The stimulator 200 and/or elements thereof could have dimensions specified to facilitate implantation into a body proximate a target tissue, to increase the overall compliance of the stimulator 200, to facilitate use of standard surgical techniques and/or tools in the implantation of the stimulator 200, to control a shape and/or size of individual volumes of a target tissue that can be stimulated with optical and/or electrical stimuli emitted from the stimulator 200, or to satisfy some other consideration. For example, the cylindrical stimulator 200 could have an outer circumference between 1 and 2.5 mm (e.g., 1.2mm), the length of the electrodes 220 (or other elements arranged between the transparent segments 235) could be between 2mm and 5 mm (e.g., 3mm), the length of the transparent segments 235 could be between 3mm and 6mm (e.g., 4mm) and the overall length of the stimulator 200, including the lead 210, could be between 25cm and 90cm (e.g., 50cm). The light emitters 230 could be very small, with a largest dimension less than 1mm (e.g., 1mm x 0.5mm x 0.5mm).

In order to provide current/voltage to the light emitters 230 and electrodes 220, wiring can be routed along the length of the lead 210 and stimulator 200 to the light emitters 230 and electrodes 220. Such wiring can be routed within one or more spaces or cavities within the stimulator 200. It may also be desirable to provide a central lumen running along the length of the stimulator 200 into which a stylet or other guide could be emplaced during implantation, in order to stiffen the stimulator 200 and guide it into position. Such a stylet could also allow the stimulator 200 to have a higher compliance, since the stiffness of the stylet could facilitate implantation of the stimulator 200 (e.g., penetration of intervening tissues).

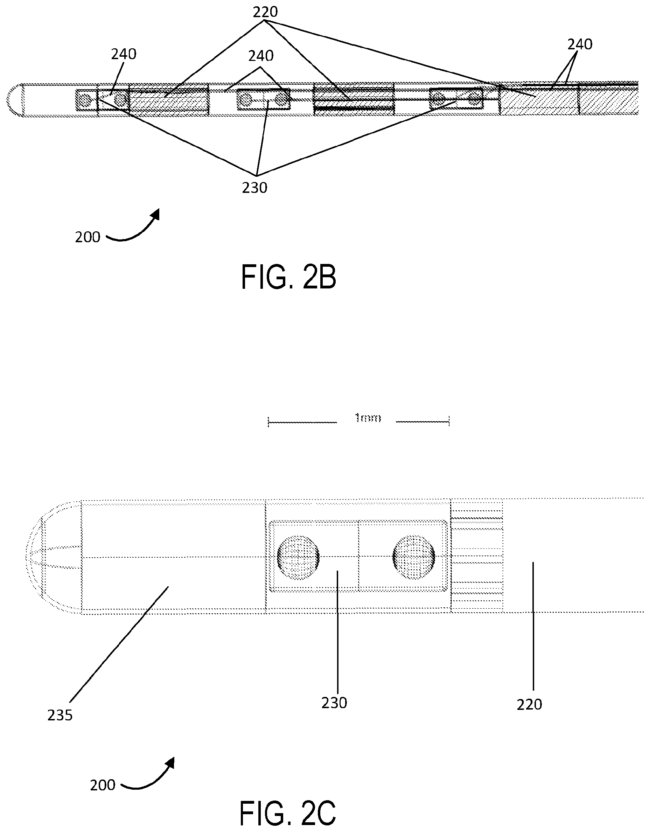

Figures 2B and 2C illustrate, in close-up view, elements of an end of the stimulator 200. Exemplary dimensions are provided for scale; in practice, other dimensions are possible. Wires 240

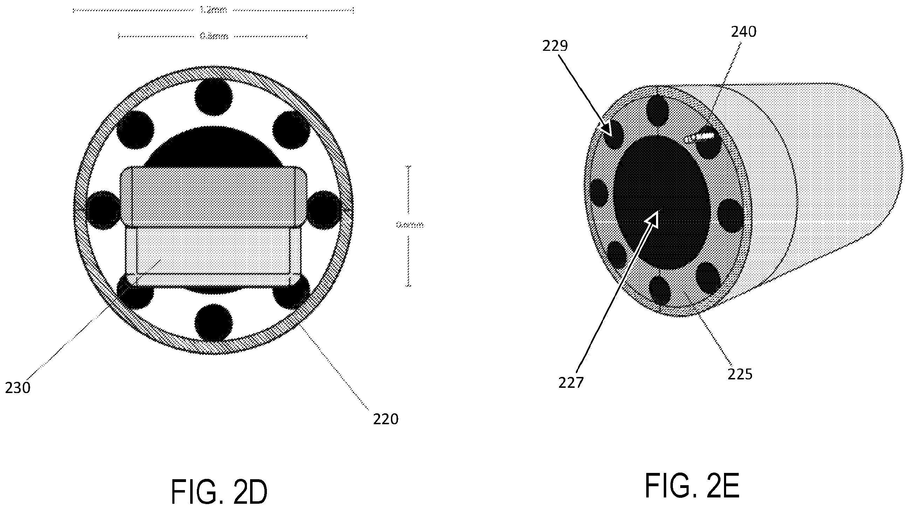

Figures 2D and 2E illustrate aspects of an example electrode 220 (or intervening segment of a stimulator that lacks electrodes) that is disposed between the transparent segments 235 of the stimulator 200. Exemplary dimensions are provided for scale; in practice, other dimensions are possible. A transverse element 225 is disposed within the electrode 220 segment. The transverse element 225 may be part of the electrode 220 (e.g., formed along with the electrode segment via casting or machining, fused to the electrode via a weld or solder) or may be a separate component disposed within the electrode 220 (e.g., via press fitting, adhesives, etc.). The transverse element 225 may be composed of conductive or non-conductive materials.

The transverse element 225 includes a central aperture 227. The central aperture 227 could be sized and/or shaped (e.g., with one or more keys or other alignment features) to accept a stylet that could be inserted into the stimulator 200 in order to facilitate implantation of the stimulator 200.

The transverse element 225 also includes a number of peripheral apertures 229 disposed around the central aperture 227. Wires (e.g., 240) can pass through the peripheral apertures 229 to provide current/voltage to the electrodes 220 and/or light emitters 230 of the stimulator 200. Routing the wires through the peripheral apertures 229 facilitates assembly of the stimulator 200, provides organization to the wiring, and also protects the wiring from damage when a stylet or other elements are inserted into/removed from the central aperture 227.

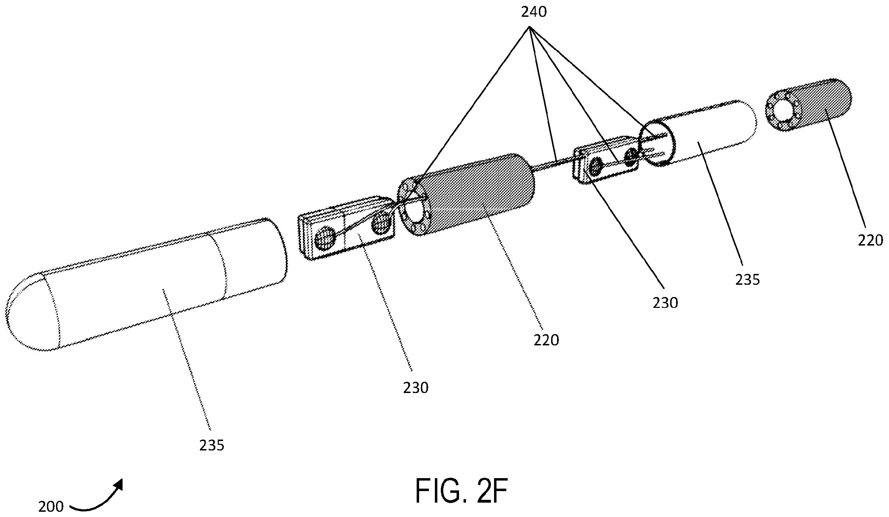

Figure 2F illustrates elements of the stimulator 200 in exploded view, showing how elements of the stimulator 200 are stacked and/or pass through each other.

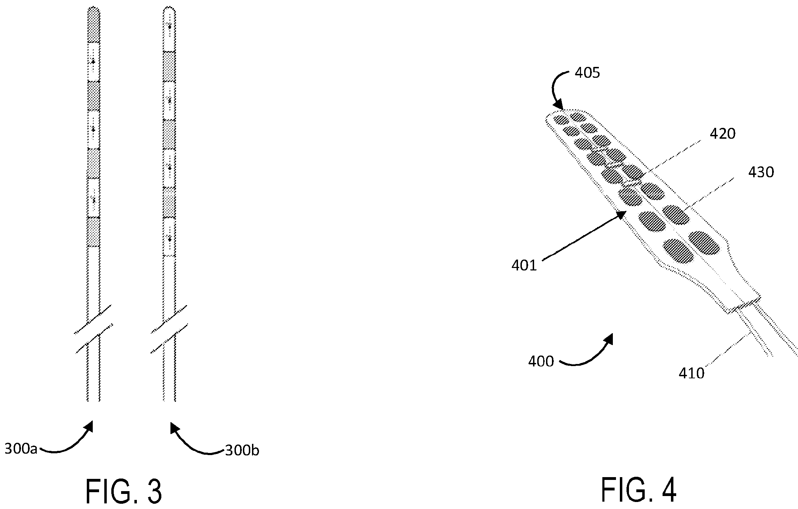

As shown in Fig. 2A, a stimulator as described herein can include a number of light emitters disposed in corresponding fully or partially transparent segments of the stimulator and alternating with non-transparent segments (which may be used as electrodes and/or which may include electrodes). These alternating segments could extend to the end of the stimulator (as shown in Fig. 2A) or could end at some point before the end of the stimulator. Where the alternating segments continue to the end of the stimulator, the alternating pattern of light emitter segments and non-light-emitter segments (e.g., electrode segments) could terminate with a light emitter segment or with a non-light-emitter segment. Fig. 3 shows a first stimulator 300a wherein the alternating pattern terminates with a non-light-emitter (e.g., electrode) segment. Fig. 3 also shows a second stimulator 300b wherein the alternating pattern terminates with a light emitter segment.

As noted above, the optical or combined optical and electrical stimulators described herein could have a variety of geometries according to an application (e.g., the cylindrical, catheter-type geometry depicted in Fig. 2A-C and Fig. 3). In another example, such a stimulator could have a "paddle" geometry, with a stimulator body being substantially flat. Such a paddle could be elongated in one of the directions along which is it substantially flat.

Fig 4. shows an example of a paddle-type stimulator 400. The stimulator 400 receives voltages/currents via a lead 410. A number of light emitters 420 and electrodes 430 are disposed on a flat surface 401 of the stimulator 400. The electrodes 430 are arranged in pairs, with the shape and location of electrodes of each pair being mirrored across a midline 405 that extends along a long axis of the paddle-type stimulator 400. The light emitters 420 alternate, along the long axis of the stimulator 400, with pairs of the electrodes 430.

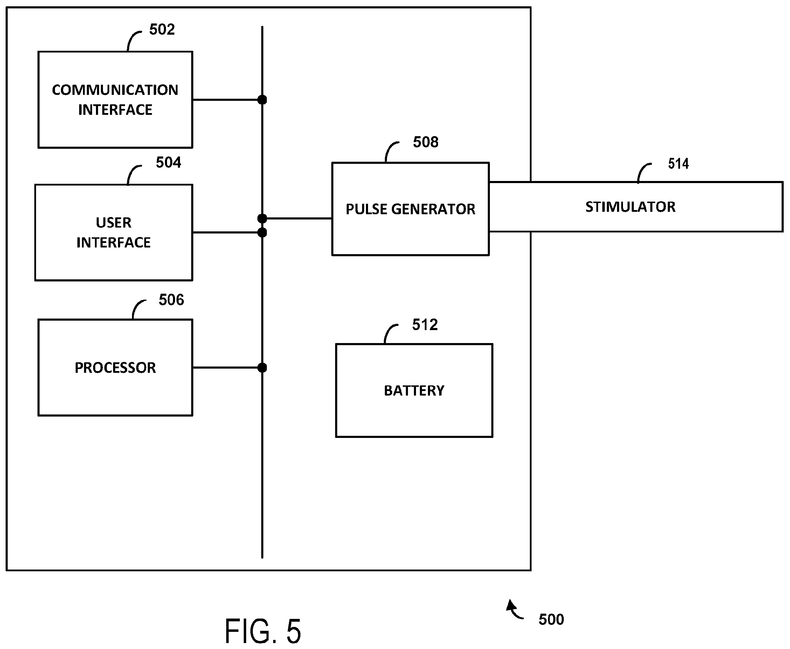

Note that the stimulator 400 depicted in Fig. 5 is a non-limiting example of a paddle-type of stimulator as described herein. Such a stimulator could have more or less of the depicted elements, arranged in similar or different patterns. For example, a stimulator could include multiple rows of light emitters (e.g., a pair of rows) arranged along the long axis of the stimulator. Such a stimulator could lack electrodes entirely, and only be configured to provide optical stimulation. Such a stimulator could have a rounded, square, or other non-elongated shape. Other variations of paddle-type stimulator are possible.

### III. Example Systems

Figure 5 illustrates an example system 500 that may be used to implement the methods described herein. By way of example and without limitation, system 500 may be a fully implantable stimulator system. In such an example, the system 500 could be provided as a single pre-assembled device (e.g., with joins between elements hermetically sealed) or as a kit of elements (e.g., a housing containing a pulse generator, battery, and other elements, a stimulator, and/or cabling to connect the stimulator to the housing). Alternatively, the system 500 could be configured to be partially implanted, e.g., such that a stimulator and/or cabling coupled thereto passing outside of a patient's body (e.g., transcutaneously) to connect with a stimulus generator unit location outside the body. Such a system could be employed to provide a temporary intervention using the stimulator and/or to determine the proper location and/or stimulus profile for the stimulation, after which the stimulator could be detached and attached to an implanted pulse-generator unit, thereby resulting in a system that is fully implanted.

As shown in Figure 5, computing system 500 may include a communication interface 502, a user interface 504, a processor 506, pulse generator 508, a stimulator 514, and a battery 512.

Where the communication interface 502 is present, it may function to allow system 500 to communicate, using analog or digital modulation of electric, magnetic, electromagnetic, optical, or other signals, with other devices, access networks, and/or transport networks. Thus, communication interface 502 may facilitate receiving programming updates, updated stimulation profiles or other information related to treatment using the system 500, or other operational data. Additionally or alternatively, the communication interface 502 may facilitate receiving commands from a user-controlled device (e.g., a cell phone or other generic device adapted to transmit such commands and/or a purpose-built device for transmitting such commands). Such commands could include commands to stop or start stimulation or some other therapy, to adjust an amplitude or other parameter of such operation (e.g., to increase or decrease an amplitude of electrical and/or optical stimulus generated by the device), to switch between therapeutic modes, or to control some other aspect of operation of the system 500.

Where the user interface 504 is present, it may function to allow system 500 to interact with a user, for example to receive input from and/or to provide output to the user. Where the system 500 is wholly implanted, this could include operating a mechanical switch, magnetic field sensor or magnetic switch, or other elements to receive inputs from a user to modify some aspect of operation of the system 500. Where the system 500 only partially implanted (e.g. only the stimulator 514 is located within a patient's body), user interface 504 may include input components such as a keypad, keyboard, touch-sensitive or presence-sensitive panel, computer mouse, trackball, joystick, microphone, and so on. User interface 504 may also include one or more output components such as a display screen which, for example, may be combined with a presence-sensitive panel. The display screen may be based on CRT, LCD, and/or LED technologies, or other technologies now known or later developed. User interface 504 may also be configured to generate audible output(s), via a speaker, speaker jack, audio output port, audio output device, earphones, and/or other similar devices.

Processor 506 may comprise one or more general purpose processors - e.g., microprocessors - and/or one or more special purpose processors - e.g., digital signal processors (DSPs), graphics processing units (GPUs), floating point units (FPUs), network processors, tensor processing units (TPUs), or application-specific integrated circuits (ASICs). In some instances, special purpose processors may be capable of determining the timing, amplitude, shape, and other properties of pulses of electrical and/or optical stimulus provided via one or more electrodes and/or light emitters of the stimulator 514.

The stimulator 514 may be any type of optical and/or combined electrical and optical stimulator as described herein. The pulse generator 508 include timers, current sources, voltage sources, digital to analog converters, amplifiers, fuses or other current or voltage limiting elements, blocking capacitors, and/or any other circuitry or components configured to drive the electrode(s) and/or light emitter(s) of the stimulator 514 to provide optical and/or combined electrical and optical stimulation as described herein. The battery 512 provides power to the system 500 and may be sized to provide a desired level and amount of stimulus for at least a specified period of time, after which point the system 500 and/or components thereof (e.g., the battery 512, a pulse generator unit that includes the battery 512) may be replaced surgically.

### IV. Conclusion

It should be understood that arrangements described herein are for purposes of example only. As such, those skilled in the art will appreciate that other arrangements and other elements (e.g. machines, interfaces, operations, orders, and groupings of operations, etc.) can be used instead, and some elements may be omitted altogether according to the desired results. Further, many of the elements that are described are functional entities that may be implemented as discrete or distributed components or in conjunction with other components, in any suitable combination and location, or other structural elements described as independent structures may be combined.

While various aspects and implementations have been disclosed herein, other aspects and implementations will be apparent to those skilled in the art. The various aspects and implementations disclosed herein are for purposes of illustration and are not intended to be limiting, with the true scope being indicated by the following claims. It is also to be understood that the terminology used herein is for the purpose of describing particular implementations only, and is not intended to be limiting.

## Claims

1. An implantable stimulator (200) configured to be secured proximate to a target tissue, the implantable stimulator comprising:
an elongate cylindrical lead body (215), wherein the lead body comprises a first plurality of cylindrical segments that alternate with a second plurality of cylindrical segments (235) along a long axis of the lead body, wherein at least part of an outer surface of one of the second plurality of cylindrical segments is formed from a transparent flexible material;
a plurality of electrodes (220), wherein each electrode of the plurality of electrodes forms at least part of an external surface of a respective one of the first plurality of cylindrical segments of the lead body; and
a plurality of light emitters (230), wherein each light emitter of the plurality of light emitters is disposed within a respective one of the second plurality of cylindrical segments of the lead body such that the second plurality of cylindrical segments respectively enclose each light emitter of the plurality of light emitters, and wherein each light emitter of the plurality of light emitters is configured to emit light at a specified wavelength, wherein at least one light emitter of the plurality of light emitters (230) is configured to emit non-coherent light at the specified wavelength.

2. The implantable stimulator (200) of claim 1, wherein the transparent flexible material comprises poly(methyl methylacrylate), PMMA.

3. The implantable stimulator (200) of claim 1 or claim 2, wherein the transparent flexible material has a cylindrical shape and wherein all of the outer surface of the given one of the second plurality of cylindrical segments is formed from the transparent flexible material.

4. The implantable stimulator (200) of any preceding claim, further comprising a transverse element (225) that is transverse to the long axis of the lead body (215) and that is disposed within one of the first plurality of cylindrical segments, and wherein the transverse element has a central aperture (227) shaped to permit passage of a stylet.

5. The implantable stimulator (200) of claim 4, wherein the transverse element (225) also has a plurality of peripheral apertures (229) disposed around the central aperture (227), and wherein the implantable stimulator additionally includes a wire (240) that is connected to one of the light emitters (230) and that passes through one of the peripheral apertures.

6. The implantable stimulator (200) of any preceding claim, wherein the elongate cylindrical lead body (215) has an outer circumference between 1 millimeter and 2.5 millimeters and a total length between 25 centimeters and 90 centimeters, wherein at least one of the first plurality of cylindrical segments has a length between 2 millimeters and 5 millimeters, and wherein at least one of the second plurality of cylindrical segments has a length between 3 millimeters and 6 millimeters.

7. The implantable stimulator (200) of any preceding claim, wherein a first light emitter and a second light emitter of the plurality of light emitters (230) are located proximate to each other within the elongate cylindrical lead body (215) such that light emitted from the first light emitter constructively and destructively interferes with light emitted from the second light emitter, thereby enhancing a magnitude of optical excitation of a focal volume within a portion of the target tissue while reducing a magnitude of optical excitation of one or more other volumes within the portion of the target tissue when the implantable stimulator is proximate to the target tissue.

8. The implantable stimulator (200) of any preceding claim, further comprising a controller, wherein the controller comprises one or more processors configured to perform controller operations comprising:
operating a first light emitter of the plurality of light emitters (230) to emit light at the specified wavelength to provide optical excitation to a portion of the target tissue; and
operating a first electrode of the plurality of electrodes (220) to provide electrical excitation to the portion of the target tissue while the first light emitter is emitting light.

9. The implantable stimulator (200) of any preceding claim, further comprising a controller, wherein the controller comprises one or more processors configured to perform controller operations comprising:
operating a first electrode of the plurality of electrodes (220) to provide electrical excitation to a portion of the target tissue; and
subsequent to operating the first electrode to provide electrical excitation, operating a first light emitter (230) of the plurality of light emitters to emit light at the specified wavelength to provide optical excitation to the portion of the target tissue.

10. The implantable stimulator (200) of any preceding claim, wherein the at least one light emitter comprises a superluminescent diode.

11. The implantable stimulator (200) of any preceding claim, wherein the specified wavelength comprises a wavelength that is between 600 nanometers and 700 nanometers or between 770 nanometers and 1200 nanometers.

## Patentansprüche

1. Implantierbarer Stimulator (200), der so konfiguriert ist, dass er unmittelbar neben einem Zielgewebe befestigt wird, wobei der implantierbare Stimulator umfasst:
einen länglichen zylindrischen Leitungskörper (215), wobei der Leitungskörper eine erste Vielzahl von zylindrischen Segmenten umfasst, die sich mit einer zweiten Vielzahl von zylindrischen Segmenten (235) entlang einer Längsachse des Leitungskörpers abwechseln, wobei mindestens ein Abschnitt einer Außenfläche eines der zweiten Vielzahl von zylindrischen Segmenten aus einem transparenten flexiblen Material gebildet ist;
eine Vielzahl von Elektroden (220), wobei jede Elektrode der Vielzahl von Elektroden mindestens einen Abschnitt einer Außenfläche eines jeweiligen der ersten Vielzahl von zylindrischen Segmenten des Leitungskörpers bildet; und
eine Vielzahl von Lichtemittern (230), wobei jeder Lichtemitter der Vielzahl von Lichtemittern innerhalb eines jeweiligen der zweiten Vielzahl von zylindrischen Segmenten des Leitungskörpers angeordnet ist, so dass die zweite Vielzahl von zylindrischen Segmenten jeweils jeden Lichtemitter der Vielzahl von Lichtemittern umschließt, und wobei jeder Lichtemitter der Vielzahl von Lichtemittern so konfiguriert ist, dass er Licht mit einer bestimmten Wellenlänge emittiert, wobei mindestens ein Lichtemitter der Vielzahl von Lichtemittern (230) so konfiguriert ist, dass er nicht-kohärentes Licht mit der bestimmten Wellenlänge emittiert.

2. Implantierbarer Stimulator (200) nach Anspruch 1, wobei das transparente flexible Material Poly(methylmethacrylat), PMMA, umfasst.

3. Implantierbarer Stimulator (200) nach Anspruch 1 oder Anspruch 2, wobei das transparente flexible Material eine zylindrische Form aufweist und wobei die gesamte Außenfläche des gegebenen einen der zweiten Vielzahl von zylindrischen Segmenten aus dem transparenten flexiblen Material gebildet ist.

4. Implantierbarer Stimulator (200) nach einem vorhergehenden Anspruch, ferner umfassend ein Querelement (225), das quer zur Längsachse des Leitungskörpers (215) ist und das innerhalb eines der ersten Vielzahl von zylindrischen Segmenten angeordnet ist, und wobei das Querelement eine zentrale Öffnung (227) aufweist, die so geformt ist, dass sie den Durchgang eines Mandrins ermöglicht.

5. Implantierbarer Stimulator (200) nach Anspruch 4, wobei das Querelement (225) auch eine Vielzahl von peripheren Öffnungen (229) aufweist, die um die zentrale Öffnung (227) herum angeordnet sind, und wobei der implantierbare Stimulator zusätzlich einen Draht (240) beinhaltet, der mit einem der Lichtemitter (230) verbunden ist und der durch eine der peripheren Öffnungen verläuft.

6. Implantierbarer Stimulator (200) nach einem vorhergehenden Anspruch, wobei der längliche zylindrische Leitungskörper (215) einen Außenumfang zwischen 1 Millimeter und 2,5 Millimeter und eine Gesamtlänge zwischen 25 Zentimetern und 90 Zentimetern aufweist, wobei mindestens eines der ersten Vielzahl von zylindrischen Segmenten eine Länge zwischen 2 Millimetern und 5 Millimetern aufweist und wobei mindestens eines der zweiten Vielzahl von zylindrischen Segmenten eine Länge zwischen 3 Millimetern und 6 Millimetern aufweist.

7. Implantierbarer Stimulator (200) nach einem vorhergehenden Anspruch, wobei ein erster Lichtemitter und ein zweiter Lichtemitter der Vielzahl von Lichtemittern (230) unmittelbar aneinander innerhalb des länglichen zylindrischen Leitungskörpers (215) so angeordnet sind, dass von dem ersten Lichtemitter emittiertes Licht konstruktiv und destruktiv mit von dem zweiten Lichtemitter emittiertem Licht interferiert, wodurch eine Größe der optischen Erregung eines fokalen Volumens innerhalb eines Abschnitts des Zielgewebes verbessert wird, während eine Größe der optischen Erregung von einem oder mehreren anderen Volumina innerhalb des Abschnitts des Zielgewebes verringert wird, wenn sich der implantierbare Stimulator unmittelbar neben dem Zielgewebe befindet.

8. Implantierbarer Stimulator (200) nach einem vorhergehenden Anspruch, ferner umfassend eine Steuerung, wobei die Steuerung einen oder mehrere Prozessoren umfasst, die so konfiguriert sind, dass sie Steuerungsoperationen durchführen, umfassend:
Betreiben eines ersten Lichtemitters der Vielzahl von Lichtemittern (230), um Licht mit der spezifizierten Wellenlänge zu emittieren, um einem Abschnitt des Zielgewebes optische Erregung zu liefern; und
Betreiben einer ersten Elektrode aus der Vielzahl von Elektroden (220), um dem Abschnitt des Zielgewebes elektrische Erregung zu liefern, während der erste Lichtemitter Licht emittiert.

9. Implantierbarer Stimulator (200) nach einem vorhergehenden Anspruch, ferner umfassend eine Steuerung, wobei die Steuerung einen oder mehrere Prozessoren umfasst, die so konfiguriert sind, dass sie Steuerungsoperationen durchführen, umfassend:
Betreiben einer ersten Elektrode aus der Vielzahl von Elektroden (220), um einen Abschnitt des Zielgewebes elektrische Erregung zu liefern; und
anschießend an das Betreiben der ersten Elektrode, um elektrische Erregung zu liefern, Betreiben eines ersten Lichtemitters (230) aus der Vielzahl von Lichtemittern, um Licht bei der spezifizierten Wellenlänge zu emittieren, um dem Abschnitt des Zielgewebes eine optische Erregung zu liefern.

10. Implantierbarer Stimulator (200) nach einem vorhergehenden Anspruch, wobei der mindestens eine Lichtemitter eine Superlumineszenzdiode umfasst.

11. Implantierbarer Stimulator (200) nach einem vorhergehenden Anspruch, wobei die spezifizierte Wellenlänge eine Wellenlänge umfasst, die zwischen 600 Nanometern und 700 Nanometern oder zwischen 770 Nanometern und 1200 Nanometern liegt.

## Revendications

1. Stimulateur implantable (200) conçu pour être fixé à proximité d'un tissu cible, le stimulateur implantable comprenant :
un corps de conducteur cylindrique allongé (215), le corps de conducteur comprenant une première pluralité de segments cylindriques qui alternent avec une seconde pluralité de segments cylindriques (235) le long d'un axe long du corps de conducteur, au moins une partie d'une surface externe de l'un de la seconde pluralité de segments cylindriques étant formée à partir d'un matériau souple transparent ;
une pluralité d'électrodes (220), chaque électrode de la pluralité d'électrodes formant au moins une partie d'une surface externe de l'un respectif de la première pluralité de segments cylindriques du corps de conducteur ; et
une pluralité d'émetteurs de lumière (230), chaque émetteur de lumière de la pluralité d'émetteurs de lumière étant disposé à l'intérieur d'un segment respectif de la seconde pluralité de segments cylindriques du corps de conducteur de sorte que la seconde pluralité de segments cylindriques enferme respectivement chaque émetteur de lumière de la pluralité d'émetteurs de lumière, et chaque émetteur de lumière de la pluralité d'émetteurs de lumière étant conçu pour émettre une lumière à une longueur d'onde spécifiée, au moins un émetteur de lumière de la pluralité d'émetteurs de lumière (230) étant conçu pour émettre une lumière non cohérente à la longueur d'onde spécifiée.

2. Stimulateur implantable (200) selon la revendication 1, ledit matériau souple transparent comprenant du poly(méthylacrylate de méthyle), PMMA.

3. Stimulateur implantable (200) selon la revendication 1 ou la revendication 2, ledit matériau souple transparent comportant une forme cylindrique et toute la surface externe du segment donné de la seconde pluralité de segments cylindriques étant formée à partir du matériau souple transparent.

4. Stimulateur implantable (200) selon l'une quelconque des revendications précédentes, comprenant en outre un élément transversal (225) qui est transversal à l'axe long du corps de conducteur (215) et qui est disposé dans l'un de la première pluralité de segments cylindriques, et ledit élément transversal comportant une ouverture centrale (227) formée de manière à permettre le passage d'un stylet.

5. Stimulateur implantable (200) selon la revendication 4, ledit élément transversal (225) comportant également une pluralité d'ouvertures périphériques (229) disposées autour de l'ouverture centrale (227), ledit stimulateur implantable comprenant en outre un fil (240) qui est connecté à l'un des émetteurs de lumière (230) et qui passe à travers l'une des ouvertures périphériques.

6. Stimulateur implantable (200) selon l'une quelconque des revendications précédentes, ledit corps de conducteur cylindrique allongé (215) comportant une circonférence externe comprise entre 1 millimètre et 2,5 millimètres et une longueur totale comprise entre 25 centimètres et 90 centimètres, au moins l'un de la première pluralité de segments cylindriques comportant une longueur comprise entre 2 millimètres et 5 millimètres, et au moins l'un de la seconde pluralité de segments cylindriques comportant une longueur comprise entre 3 millimètres et 6 millimètres.

7. Stimulateur implantable (200) selon l'une quelconque des revendications précédentes, un premier émetteur de lumière et un second émetteur de lumière de la pluralité d'émetteurs de lumière (230) étant situés à proximité l'un de l'autre à l'intérieur du corps de conducteur cylindrique allongé (215) de sorte que la lumière émise par le premier émetteur de lumière interfère de manière constructive et destructive avec la lumière émise par le second émetteur de lumière, ce qui permet d'améliorer une amplitude d'excitation optique d'un volume focal à l'intérieur d'une partie du tissu cible tout en réduisant l'amplitude d'excitation optique d'un ou plusieurs autres volumes à l'intérieur de la partie du tissu cible lorsque le stimulateur implantable se trouve à proximité du tissu cible.

8. Stimulateur implantable (200) selon l'une quelconque des revendications précédentes, comprenant en outre un dispositif de commande, ledit dispositif de commande comprenant un ou plusieurs processeurs conçus pour réaliser des opérations de dispositif de commande comprenant :
le fonctionnement d'un premier émetteur de lumière de la pluralité d'émetteurs de lumière (230) pour émettre une lumière à la longueur d'onde spécifiée de manière à fournir une excitation optique à une partie du tissu cible ; et
le fonctionnement d'une première électrode de la pluralité d'électrodes (220) pour fournir une excitation électrique à la partie du tissu cible pendant que le premier émetteur de lumière émet de la lumière.

9. Stimulateur implantable (200) selon l'une quelconque des revendications précédentes, comprenant en outre un dispositif de commande, ledit dispositif de commande comprenant un ou plusieurs processeurs conçus pour réaliser des opérations de dispositif de commande comprenant :
le fonctionnement d'une première électrode de la pluralité d'électrodes (220) pour fournir une excitation électrique à une partie du tissu cible ; et
après le fonctionnement de la première électrode pour fournir une excitation électrique, le fonctionnement d'un premier émetteur de lumière (230) de la pluralité d'émetteurs de lumière pour émettre une lumière à la longueur d'onde spécifiée de manière à fournir une excitation optique à la partie du tissu cible.

10. Stimulateur implantable (200) selon l'une quelconque des revendications précédentes, ledit au moins un émetteur de lumière comprenant une diode superluminescente.

11. Stimulateur implantable (200) selon l'une quelconque des revendications précédentes, ladite longueur d'onde spécifiée comprenant une longueur d'onde qui est comprise entre 600 nanomètres et 700 nanomètres ou entre 770 nanomètres et 1200 nanomètres.
